# EUROPEAN PATENT APPLICATION

(11) **EP 2 987 408 A1**
(43) Date of publication of application: **24.02.2016**
(21) Application number: 14181684.3
(22) Date of filing: 20.08.2014
(51) Int. Cl.: A01N 59/12, A01N 59/24, A01N 25/22, A01P 1/00, A61K 33/18, A61K 47/48, A61K 9/00, A61K 8/20, A61K 8/22, A23C 7/00

(54) **Iodophor composition with improved stability in the presence of organic material**

(71) Applicant: National University of Ireland, Galway, Galway (IE)
(72) Inventor: O'Flaherty, Vincent, Moycullen, Co. Galway (IE); McCay, Paul, Newcastle, Co. Galway (IE)
(74) Representative: Gates, Marie Christina Esther

(57) **Abstract**

The invention is concerned with a pharmaceutical and industrial iodophor preparation, its synthesis and potential applications. The compound has predictable antimicrobial activities. Furthermore, this iodophor is much more stable in the presence of organic material than traditional iodophors. The compositions release free iodine when in solution, which provides the antimicrobial activity.

## Description

### Background of the Invention

This invention is concerned with a pharmaceutical and industrial iodophor preparation, its synthesis and potential applications. The compound has predictable antimicrobial activities. Furthermore, this iodophor is much more stable in the presence of organic material than traditional iodophors. lodophors are iodine based compounds complexed with a stabilization agent. They release free iodine when in solution, which provides the antimicrobial activity. They are typically prepared by mixing iodine with the stabilization agent, with heating.

Iodophors exert antimicrobial activity as the free iodine interacts with key bacterial proteins causing bacteriostatic or bacteriocidal effects. They have a potential wide-range of uses, from cleaning and sterilising industrial or medical equipment, to wound treatment (after or before infection as a prophylactic). Diluted iodophors are used extensively in the dairy industry to clean machinery, minimising the spread of potential pathogenic organisms. Iodophors are not only antimicrobial but strongly antiviral and antifungal. Their use requires very little post-rinsing, unlike hypochlorite based bleaching compounds. Their use is common in aquatic tanks as a method of minimising excess growth of bacteria or parasitic organisms. They can be used to kill causative organisms associated with acne and skin conditions, as well as a treatment for the flare up of herpes infections (virus). Iodophors have been used for many years and are generally recognised as safe compounds. Based on a review of the available toxicology data, the US EPA concluded that "iodine and iodophor complexes are of very low toxicity by the oral, dermal, and inhalation routes of exposure". The most common iodophor is polyvinylpyrrolidone iodine (known as PVP-iodine, PVP-I, or povidone, and by its brand name Betadine, or Wokadine). The PVP polymer also serves to reduce the potential toxicity effects of iodine. There havebeen very few reported resistance/tolerance characteristics in bacteria to iodine-mediated killing. This is more than likely due to the indiscriminate targeting by the iodophor of various bacterial proteins within the cell. Such tolerances/resistance phenotypes would require multiple, and simultaneous beneficial mutations in the bacterial genome.

The use of PVP-I has overcome some of the disadvantages of tinctures of iodine and Lugol's solution (a solution of iodine with iodide ions) as regards long term storage, free vs total iodine and toxicity issues etc. However, the commonly used iodophors such as PVP-I are very unstable in the presence of organic material. On dissolving PVP-I in water, the compound exerts significant antimicrobial activity, but minimal antimicrobial activity in the presence of organic matter. For example, the minimum inhibitory concentration (MIC) of PVP-I in water for *E. coli* ATCC 25922 is <1 mg L⁻¹. On repetition of the test in an organic rich growth medium (such as lysogeny broth), the MBC value is recorded at >500 mg L⁻¹. This discrepancy in MBC values indicates the limitations of traditional iodophors. Use of such a concentration in many environments would be practically difficult, prohibitively expensive and potentially toxic.

In the dairy industry, PVP-I is regularly used to clean teats of cows before and after milking to kill bacteria present. However, if the PVP-I comes into contact with milk, activity is quickly diminished. The same pattern occurs in other industries, such as baking etc, as food residue left on machinery can interact with the PVP-I and halt its antimicrobial effect.

An object of the present invention is to provide a novel iodophor which is considerably more stable in the presence of organic materials than traditional iodophors. A further object is to provide iodophor producing compositions, particularly compositions which can be used for sterilising environments with a high organic matter load.

### Brief Description of the Drawings

Figure 1 : Effect of Lugol's solution and novel iodophor on milk colour (doubling dilutions left to right starting with 500 mg L⁻¹)

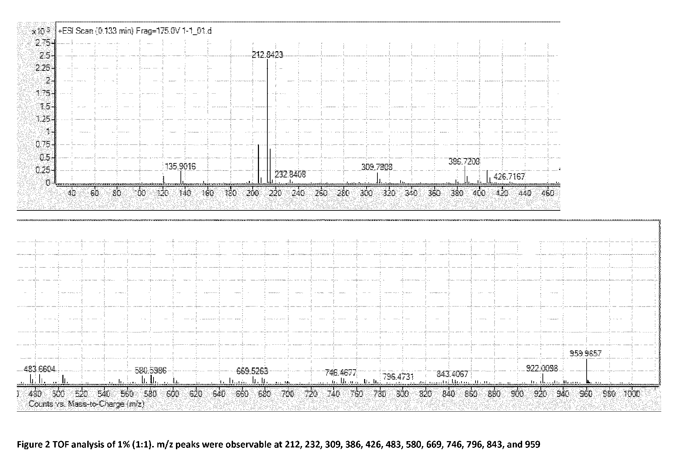

Figure 2 Time-of-flight mass spectrometry (TOF-MS) analysis of 1% (1:1). m/z peaks were observable at 212, 232, 309, 386, 426, 483, 580, 669, 746, 796, 843, and 959

### Detailed Description of the Invention

According to the present invention there is provided an iodophor-producing composition comprising iodide or iodate ions, thiocyanate ions and an oxidising agent.

The source of the iodide/iodate may be potassium iodide, sodium iodide, lithium iodide, caesium iodide, hydrogen iodide, rhodium iodide, or a slow-release form of iodide, potassium iodate or sodium iodate.

The source of the thiocyanate ions may be potassium thiocyanate, lithium thiocyanate, caesium thiocyanate, hydrogen thiocyanate, rhodium thiocyanate, a slow-release form of thiocyanate, or a cyanate compound such as potassium cyanate or thiocyanogens.

The oxidising agent may be hydrogen peroxide or potassium permanganate, sodium peroxide, lithium peroxide, peroxide releasing percarbonates, peroxide releasing citric acid or vitamin C, peroxide salts including barium oxide, sodium perborate, a hydrogen peroxide-urea adduct, oxygen releasing pseudo-peroxides including superoxides, dioygenals, ozones, ozonides, organic peroxides including peroxy acids, acyl halides and aliphatic peroxides.

In some embodiments potassium iodate may serve as both the oxidising agent and the source of the iodate/iodine ions.

The invention also provides an iodophor comprising an lodo-thiocyanate complex containing an oxygen, such as I₂OHS(CN)₂.

The novel iodophor can be prepared by the mixing of a source of iodide ions (such as potassium iodide), a source of thiocyanate ions (such as potassium thiocyanate) and an oxidising agent, such as hydrogen peroxide in an aqueous environment. A strongly antimicrobial compound can be prepared by the mixing of 1% w/v potassium thiocyanate (KSCN), 1% w/v potassium iodide (KI), and 1% w/v hydrogen peroxide (H₂O₂). More dilute or concentrated solutions of iodophor can be prepared by the increase or decrease in starting components. Likewise, a concentrated stock can be prepared, and diluted accordingly when required.

In this aqueous environment, the iodide and thiocyanate are simultaneously oxidised, and form an iodo-thiocyanate complex containing an oxygen atom. Further oxidation can occur, producing I₂, I₃, I₄, and I₅ species, increasing in molecular weight.

Use of hydrogen peroxide is advantageous in that there are no significant breakdown or toxicity issues as it is cleaved to form oxygen and water. Other oxidising agents can be used, for example potassium permanganate, though they do add unwanted colour and impurities to the environment. Alternatively, potassium iodate (KIO₃, 1% w/v) can serve as both the oxidising agent and iodine source as an alternative to the combination of peroxide and iodide. The reaction between it and thiocyanate proceeds at a slower rate (taking almost 24 hours at the concentrations described herein, though the reaction can be quickened up by addition of hydrogen peroxide). Importantly the iodate based reaction requires a concentration above 1% w/v of the components to produce significant antimicrobial activity. Using the iodide/thiocyanate/peroxide model, the solution can be made using much lower (0.001-0.01 % w/v) or higher concentrations and the antimicrobial activity exerted will have a linear relationship to concentration, unlike the iodate model. This is surprising. The iodophor, if prepared in an aqueous environment, can be buffered to a pH if required. It is stable in the presence of numerous salts, and at a relatively large temperature range if in a sealed environment. The iodophor can be prepared as a hydrogel (using for example 1% w/v sodium polyacrylate). This lends itself to use as a gel for burn/skin complaints.

A 5-20 ml volume of 0.5-2% w/v prepared iodophor would be capable of infusion into the udder of a mastitic ruminant animal and killing the causative organism. The treatment regimen could mirror that of antibiotic treatments (once or twice a day, for 2-10 days for a mastitic animal).

Because of the wide-ranging antimicrobial activity spectrum exerted by iodophors, the compound would be capable of killing a wide variety of bacterial (including Gram-positive, Gram-negative, antibiotic-resistant bacteria, fungal strains, and viruses).

Dilute forms (<0. 1% w/v) of the novel iodophor could be used to treat minor cuts or abrasions. More concentrated forms of the same iodophor could be used to treat more serious skin infections (~0.5 % w/v). A nebulised spray version of the iodophor may be suitable for use to treat lung infections where antibiotic usage is commonly unsuccessful. Such a delivery mechanism would also have a potential in surgeries where open wounds occur as a means of prophylactic.

Concentrated forms (> 1% w/v) could be used to clean/sterilise or decontaminate inanimate materials such as food processing equipment, steel bowls, surgical equipment etc.

Although it is preferential that simple ionic salts such as potassium or sodium variants of iodide and thiocyanate be used, other forms may also be used including, but not limited to sodium iodide, potassium iodide, lithium iodide, caesium iodide, hydrogen iodide, rhodium iodide, or by the oxidation of elemental iodine and as a source of thiocyanate; potassium thiocyanate, lithium thiocyanate, caesium thiocyanate, hydrogen thiocyanate, rhodium thiocyanate, , or a cyanate compound such as potassium cyanate or thiocyanogens.

Likewise, the use of hydrogen peroxide as an oxidising solution is preferable as it is non-toxic at required concentrations (<3% w/v) and leaves no significant residues. Alternative, less preferential, oxidising agents may be used including, but not limited to sodium peroxide, lithium peroxide or peroxide releasing percarbonates, peroxide releasing citric acid or Vitamin C, peroxide salts including barium oxide, sodium perborate, an hydrogen peroxide-urea adduct, oxygen relasing pseudo peroxides including superoxides, dioygenals, ozones, and ozonides, organic peroxides including peroxy acids, acyl halides, aliphatic peroxides.

The iodophor can be prepared as a gel, an emulsion, an oil, a hydrogel as suits the use. The compound can be lyophilized and stored as a dried version to prolong storage. The compound can be added to bandages as a dried (and subsequently wetted) or wet product as a means to prevent or kill bacterial infection.

A ratio range of 0.5:1 - 2:1 of iodide to thiocyanate (by weight) is important in producing significant quantities of free available iodine with the oxygenated thiocyanate stabiliser. A more preferable ratio is 0.8:1 - 1.2:1. A more preferable option again is a 1:1 ratio of thiocyanate to iodide. For example; 1,000 mg L⁻¹ each of potassium iodide and potassium thiocyanate, in the presence of sufficient hydrogen peroxide acting as an oxidising agent 800-1200 mg L⁻¹.

A ratio range of 0.25:1-4:1 of hydrogen peroxide to iodide/thiocyanate (by weight) is sufficient in allowing the simultaneous oxidation and thus production of the iodophor. A more preferable option of hydrogen peroxide to iodide/thiocyanate is 0.5:1- 2:1. A more preferable option again is a 1:1 ratio of hydrogen peroxide to iodide/thiocyanate. At ratios of 2:1 or greater of hydrogen peroxide to iodide/thiocyanate, the compound is less stable due to pH changes, and can result in the formation of undesirable CN and SO₄ as the thiocyanate SCN bonds are broken. At a range of <1:1 of hydrogen peroxide to iodide/thiocyanate, activity is reduced as insufficient oxidation occurs.

Percarbonates are dessicated compounds that release hydrogen peroxide on mixing with an aqueous environment. As such, sodium (and other) percarbonates could act as a source of hydrogen peroxide. A simple calculation based on the available hydrogen peroxide from the percarbonate would allow a simple swap of the liquid peroxide for a dried percarbonate. Furthermore, other compounds that oxidise and release free O could be used on determination of the free O content likely to be achieved. Simple oxidoreductase enzymes are capable of reacting with monosaccharide sugars to produce a source of hydrogen peroxide. Such a system could be used by titreing the correct level of hydrogen peroxide produced in the environment.

It is surprising that the use of a thiocyanate provides such stabilisation in the presence of organic material. SCN is a linear compound. It isn't one particular atom providing the basis for the characterisation. Allyl isothiocyanate was inefficient in producing the same results suggesting that the reaction is very dependent on access to the atoms. A number of sulfer (S) containing compounds including a variety of sulfates, thiols, and thio-based compounds such as methionine did not allow production of an organic-material-stable iodophor in the same manner. Likewise, a number of cyanate-based compounds such as sodium dichloroisocyanturate or potassium cyanate were not efficient in allowing the production of an organic-material-stable iodophor either. This makes the result of using thiocyanate all the more surprising and novel.

Depending on the degree of oxidation and the relative concentrations, a number of potential iodo-thiocyanate compounds are produced and release, from single iodine, right up to seven iodine atoms per molecule. Such compounds are ISCN, I₂SCN, I₂(SCN)₂, IOH(SCN)₂, I₃OH(SCN)₂, I₃OH(SCN)₃, I₄(SCN)₄OH, I₅(SCN)₅.

The iodophor of the invention is produced in solution by the simultaneous oxidation of iodide/iodate ions (to allow formation of iodine) and thiocyanate ions, which serve to stabilise the resulting antimicrobially active iodine. The resulting iodophor is an iodo-thiocyanate complex class compound. It can be prepared as a dilute or more concentrated preparation depending on requirements. The novel iodophor can be prepared *in situ* by the mixing of iodide/iodate and thiocyanate ions (in the presence of a suitable oxidising agent, for example hydrogen peroxide), or be prepared *ex situ* and applied/added accordingly. It exerts strong antimicrobial activity in water based environments like other iodophors. However, the MIC for E. coli ATCC25922 in lysogeny broth (LB) remains below 10 mg L⁻¹, unlike that for PVP-I (500 mg L⁻¹).

This novel iodophor has many potential uses as an antimicrobial agent where traditional iodophors have been used, namely; wound/burn treatment, an anti-viral topical treatment, machinery sterilant, skin wipe, anti-fungal topical agent. Furthermore, the novel iodophor is suited to a number of potential uses that traditional iodophor compounds would be unsuited for due to the presence of organic material. Examples of such environments include as a mastitis treatment for ruminant animals where the compound is used an alternative to antibiotics in the udder with milk present. A further example is for use to 'clean' untreated milk for eradication of the causative organism of Johne's disease, which can be transferred from mother to calf in the milk. The novel iodophor could be used to extend the shelf-life of milk in areas without sufficient refrigeration. This would not be possible with traditional iodophors due to quick dissipation of the antimicrobial activity on contact with milk. Furthermore, the novel iodophor is more suited to traditional applications as it would be less susceptible to dissipation by any present organic material (sloughed cells in a wound, organic debris on milking machinery etc).

### Examples of Compositions

1 A general wash/ antimicrobial composition containing 1-100,000 mg L⁻¹ of the iodophor for use as an antimicrobial topical ointment/cream/gel/solution.
2 A general wash/ antimicrobial composition containing 10-10,000 mg L⁻¹ of the iodophor for use as an antimicrobial topical ointment/cream/gel/solution.
3 A composition containing 10-10,000 mg L⁻¹ of the iodophor for use as an inhaled antimicrobial therapy using a nebulised solution
4 A bandage dipped in or containing 10-10,000 mg L⁻¹ of the iodophor for use as an antimicrobial bandage.
5 A composition for the treamtent of mastitis in ruminant animals containing 10-10,000 mg L⁻¹ of the iodophor as an intramammary infusion for once or twice daily treatment
6 An extemporaneous antimicrobial composition for daily, or twice daily, treatment is made by means of intra-mammary devices containing components capable of producing 10-10,000 mg L⁻¹ of iodophor *in situ* as described above (iodide or iodate anion at between 10-10,000 mg L⁻¹, thiocyanate ion at between 10-10,000 mg L⁻¹ and an oxidising solution (if required) such as hydrogen peroxide at between 10-1,000 mg L⁻¹).
7 An extemporaneous antimicrobial (and anti-biofilm) composition general wash containing components capable of producing 10-1,000 mg L⁻¹ of iodophor *in situ* as described above (iodide or iodate anion at between 10-10,000 mg L⁻¹, thiocyanate ion at between 10-10,000 mg L⁻¹ and an oxidising solution (if required) such as hydrogen peroxide at between 10-10,000 mg L⁻¹) for any purposes as described in previous examples.
8 A composition as described in examples 1-7 wherein the oxidising solution is selected from a list described elsewhere in this text including, but not limited to, hydrogen peroxide, and present in sufficient concentrations to allow oxidation of the thiocyanate and iodide (or iodate) ions.

The pharmaceutically effective carrier is water, saline, an emulsion, a gel or a hydrogel.

The composition may be adapted for delivery by means of a dampened bandage.

The composition may be adapted for use as an antimicrobial nasal rinse, as an antimicrobial mouth-wash, an antifungal wash, as a disinfectant, added to a bandage or poultice for the treatment of wound or burn infections, or nebulised in the form of a spray for the treatment of bacterial or fungal infection of the human or animal lung.

The invention also provides a medical device coated with the compositions as described above, and a bandage or poultice impregnated with the compositions.

A composition described in any previous examples wherein it is supplemented to contaminated milk as an antimicrobial agent at concentrations of 10-10,000 mg L⁻¹ for the eradication of bacteria such as *Mycobacterium avium paratuberculosis.*

A composition described in any previous examples wherein it is supplemented to contaminated environments as an antimicrobial agent at concentrations of 10-10,000 mg L⁻¹ for the eradication of fungi, parasites, unicellular organisms, virsues, or bacterial/fungal spores.

A more stable form of the iodo-thiocyanate iodophor could be produced with the introduction of PVP polymer to the compound. Importantly, mixing thiocyanate with an iodine-containing PVP-I did not serve to change the structure of the existing PVP-I, demonstrating that the novel iodophor cannot be prepared in this manner.

### Example 9

A 1L batch of iodophor is prepared by the addition of 10 g potassium iodide, 10 g potassium thiocyanate to ca.950 ml of water. When the salts have dissolved fully, 33.33 ml of 30% hydrogen peroxide is added to the solution. The solution is then topped up to 1,000 ml with water. The solution can be pH adjusted and buffered to 5.5 if necessary. This solution is henceforth referred to as 1% (1:1) mixture, the ratio referring to the weight of the peroxide added to the weights of thiocyanate and iodide salts used.

An alternative to the use of hydrogen peroxide is to use ca.33g of sodium percarbonate (with 30% available hydrogen peroxide) and allowed dissolve fully to permit release of peroxide.

As a further alternative, a 1L batch of the iodophor is prepared by the addition of 10g potassium iodate and 10 g potassium thiocyanate to ca.950 ml. The solution is then topped up to 1,000 ml with water and left for 24 hours to allow the reaction to occur. The solution can then be pH adjusted and buffered to 5.5 if necessary.

The novel iodophor could further be complexed with PVP polymer such that it would possess the advantages of both PVP-I in addition to the stability in the presence of organic material of the iodophor described in the examples.

A suitable glucocorticoid could be supplemented to intramammary infusions destined for mastitic animals, or in nebulised/general wash form destined for the treatment or prevention of lung infections to help with potential local tolerance issues. The steroid could be (but not limited to) hydrocortisone/cortisol, prednisolone, or prednisone present at between 5 and 50 mg per dose.

### Results

### Minimum Inhibitory Concentrations

The efficacy of the novel 1% (1:1) as produced as described in Example 9 was tested against a bank of microorganisms associated with skin, mastitis and lung infections to determine its activity. To perform such efficacy testing, minimum inhibitory concentrations (MIC) tests were carried out using the micro-broth dilution method wherein doubling dilutions of the compound are added to wells containing the test organism (10⁵⁻⁶ colony forming units ml⁻¹) in a growth medium. The optical density of the well contents are then recorded over 24 hours. An increase in optical denisty indicates growth of the test organism. If the bacteria failed to grow over 24 hours at 37 °C in a particular well, the concentration therein was deemed inhibitory. The lowest concentration required to inhibit the growth is known as the MIC. For tests involving no growth medium (i.e. water and milk), an aliquot from the well was subcultured to fresh nutrient agar (NA). The corresponding compound concentration was deemed inhibitory if bacteria subsequently failed to grow from the sampled aliquot.

As is clear from Table 1, the iodophor (1%, 1:1) is effective at inhibiting each of the organisms (including antibiotic-resistant organisms) at concentrations of less than 30 mg L⁻¹. This level of activity is approaching antibiotic levels of activity at low concentrations. As a comparison, PVP-I and Lugol's iodine were unsuccessful at eliminating the same bacterial strains below concentrations of 500-1,000 mg L⁻¹. This is due to the deleterious inactiviation of the PVP-I or Lugol's-derived iodine by organic material present in the growth medium. Such differences in MICs were noted in various growth media and a milk environment (MIC of <30 mg L⁻¹ for the iodo-thiocyanate iodophor and >500-1000 L⁻¹ PVP-I/Lugol's).

**Table 1 : Minimum Inhibitory Concentration values of the iodo-thiocyanate iodophor for bacterial strains in Lysogney Broth**

| **Organism** | **MIC (mg/L)** |
|---|---|
| *E. coli* ATCC 25922 | 10-20 |
| *Pseudomonas aeruginosa* PA01 | 10-20 |
| *P. aeruginosa* PA-29 (antimicrobial tolerant isolate Mc Cay *et al.,* 2010) | 10-20 |
| *P. aeruginosa* 9026 (cystic fibrosis isolate, antibiotic tolerant) | 10-20 |
| *P. aeruginosa* 9027 (cystic fibrosis isolate, antibiotic tolerant) | 10-20 |
| *P. aeruginosa* 9028 (cystic fibrosis isolate, antibiotic tolerant) | 10-20 |
| *P. aeruginosa* 9029 (cystic fibrosis isolate, antibiotic tolerant) | 10-20 |
| *Staphylococcus aureus* 15676 | 15-30 |
| *S. aureus* BH1CC (MRSA, oxacillin resistant hospital isolate) | 15-30 |
| *S. eipdermidis* 1457 (oxacillin resistant hospital isolate) | 15-30 |
| *S. epidermidis* C57 | 15-30 |
| *Micrococcus luteus* | 10-20 |
| *Dermacoccus nishinomiyaensis* | 10-20 |
| *Strep. agalactiae* (isolate from mastitic ruminant) | 5-10 |
| *Strep. dysgalactine* (isolate from mastitic ruminant) | 5-10 |
| *Strep. uberis* (isolate from mastitic ruminant) | 5-10 |

Comparative concentrations of 0.01-5 mg L⁻¹ of the novel iodo-thiocyanate iodophor, Lugol's solution and PVP-I were sufficient in killing the same bacterial strains in the absence of any growth medium (i.e. a saline environment), demonstrating that the difference in relevance activity is due to the organic material.

The described compositions have been shown to be bacteriocidal and not bacteriostatic. If an antimicrobial compound is removed from the environment and the bacteria/virus/fungi/yeast once again begin to proliferate, the compound would be described as bacteriostatic (i.e. stopping the cells from growing). If they didn't recover the compound would be classed as bacteriocidal (i.e. killing of cells). In tests with the novel iodophor, viable bacterial cells were not recoverable using 20 mg L⁻¹ in saline for 1 hour, even with the addition of sodium pyruvate to the saline to help bacterial recovery. Using an equivalent concentration (and a further concentration 10 times greater) of hydrogen peroxide alone resulted in the recovery of viable cells, demonstrating that the killing was not peroxide mediated.

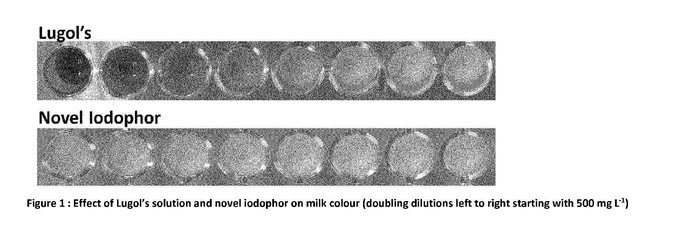

A further benefit of the novel iodophor is the relative lack of staining and colour associated with its use. Lugol's is preferable to PVP-I in terms of activity exerted in a milk environment (though about 40-fold less active than the novel iodophor). However, the concentrations required to kill the bacteria also led to a change in the appearance and colour of the milk. This is not the case with the novel iodophor. Figure 1 demonstrates the colour change in milk containing 500, 250, 125, 62.5, 31.25, 15.625, 7.8125, 3.9, and 1.95 mg L⁻¹ of each compound (left to right). It is clear that the Lugol's iodine brings about an orange and even brown colouration (down to and including at >10 mg L⁻¹) whereas the novel iodophor does not, even at 500 mg L⁻¹. This would be important when using appropriate dose concentration for mastitis or treatment of contaminated milk supplies.

A 1% (1:1) sample was examined using Time of Flight mass spectrometry (TOF) to identify the complexes responsible for antimicrobial activity. A number of peaks (indicating their mass to charge ratio - m/z) of interest were identified. Peaks at m/z 121 and 922 correspond to internal calibrants. This demonstrates that there was more than one iodo-thiocyanate complex formed during the reaction. A number of larger molecules formed as more iodine atoms attach and allow the attachment of further cyanogen molecules to attach. Peaks were observed at m/z of 212, 309, 386, 483. These m/z values would equate to a number of compounds such as ICNH(SCN), I₂SCN, I₂OH(SCN)₂, and I₃SCHN₂O. Each of these structures is an iodo-thiocyanate complex with minor substitutions and additions. Weaker peaks were identified at m/z ratios of 580, 669, and 746, and 959 indicating more iodine atoms and cyanogen molecules attaching, producing even larger complexes.

To demonstrate that further alternative oxidising agents can be employed to produce the compound instead of hydrogen peroxide, potassium permanganate was used in a disc diffusion assay; small concentrations of the components (100 µg thiocyanate/iodide/permanganate in 10 µl water) were applied to a paper disc on top of lysogeny agar freshly seeded with 10⁵ cfu/ml of E. coli ATCC 25922. Plates are incubated overnight at 37 °C and the resulting zones of inhibition are measured. A large zone indicates strong antimicrobial activity. No zone indicates a lack of perceivable antimicrobial activity. Results from the tests are presented in Table 2. Permanganate can itself exert an antimicrobial effect, but only in the absence of organic material. Therefore, any antimicrobial activity noted in samples containing permanganate is as a result of its oxidation of the thiocyanate and iodide to produce the novel iodophor. It is evident that on mixing the three compounds, strong activity was noted, but only when all three were mixed. It is clearly demonstrated therefore that alternative oxidising agents other than peroxide are capable of producing the iodophor.

**Table 2 Demonstration of iodophor production by permanganate-mediated oxidation**

| **Solution** | **Zone of inhibition** |
|---|---|
| 375 µg KMnO₄ | No zone |
| 375 µg KinO₄ + 375 µg KSCN | No zone |
| 375 µg KI + 375 µg KSCN | No zone |
| 375 µg KMnO₄ + 375 µg KI + 375 µg KSCN | 34 mm |

## Claims

1. An iodophor-producing composition comprising a source of iodide/iodate ions, a source of thiocyanate ions and an oxidising agent, which compounds are capable of reacting *in situ* to produce an iodophor.

2. A composition as claimed in claim 1 or claim 2 wherein the source of the iodide/iodate ions is selected from potassium iodide, sodium iodide, lithium iodide, caesium iodide, hydrogen iodide, rhodium iodide, potassium iodate are sodium iodate.

3. A composition as claimed in any proceeding claim wherein the source of the thiocyanate ions is selected from sodium thiocyanate, potassium thiocyanate, lithium thiocyanate, caesium thiocyanate, hydrogen thiocyanate, rhodium thiocyanate, allyl isothiocyanate, , or a cyanate compound such as potassium cyanate or thiocyanogens.

4. A composition as claimed in any proceeding claim wherein the oxidising agent is selected from hydrogen peroxide or potassium permanganate, sodium peroxide, lithium peroxide, peroxide releasing percarbonates, peroxide releasing citric acid or vitamin C, peroxide salts including barium oxide, sodium perborate, a hydrogen peroxide-urea adduct, oxygen releasing pseudo-peroxides including superoxides, dioygenals, ozones, ozonides, organic peroxides including peroxy acids, acyl halides and aliphatic peroxides.

5. A composition as claimed any preceeding claim wherein potassium iodate serves as both the oxidising agent and the iodate/iodine ion source.

6. An iodophor composition comprising the reaction products of a composition as claimed in any preceding claim when placed in an aqueous environment.

7. An iodophor composition comprising at least one oxygen, at least one iodine and at least one thiocyanogen or cyanate unit.

8. A composition as claimed any preceding claim further comprising pharmaceutically effective carriers and diluents, preferably wherein the pharmaceutically effective carrier is water, saline, an emulsion, a gel or a hydrogel.

9. The composition of any preceding claim, wherein the composition is adapted for the treatment of mastitic lactating ruminant animals,
or is adapted for use as an antimicrobial nasal rinse,
or adapted for use as an antimicrobial mouth-wash,
or adapted for use as an antifungal wash,
or adapted for use as an antimicrobial face-wash/acne cream,
or is adapted for use to treat contaminated milk or feed stuffs.

10. The composition of any preceding claim, adapted for concurrent, or sequential, delivery of the oxidising agent and iodide and thiocyanate components.

11. The composition of any preceding claim, adapted for delivery by means of an intra-mammary device, or adapted for delivery by means of a dampened bandage.

12. The composition of any preceding claim, wherein the composition is added to a bandage or poultice for the treatment of wound or burn infections.

13. The composition of any preceding claim, wherein the composition is nebulised in the form of a spray for the treatment of bacterial or fungal infection of the human or animal lung.

14. A method of reducing the bacterial contamination of milk or feedstuffs comprising administration of a composition as claimed in any of claims 1 to 9.

15. A method of reducing the bacterial load in a mammary gland comprising administration of a composition as claimed any of claims 1 to 9.
